# EUROPEAN PATENT APPLICATION

(11) **EP 3 085 703 A1**
(43) Date of publication of application: **26.10.2016**
(21) Application number: 15164169.3
(22) Date of filing: 20.04.2015
(51) Int. Cl.: C07K 1/13, A61K 47/48

(54) **Use of thiol-containing scavengers for purification of antibody-drug conjugates**

(71) Applicant: Lonza Ltd, 3930 Visp (CH)
(72) Inventor: DUCRY, LAURENT, 3960 Sierre (CH); AMBORT, DANIEL, 3900 Brig (CH)

(57) **Abstract**

The invention discloses the use of thiol-containing scavengers immobilized on solid supports to separate unconjugated drug-related impurities after the conjugation of a drug to a biomolecule.

## Description

The invention discloses the use of thiol-containing scavengers immobilized on solid supports to separate unconjugated drug-related impurities after the conjugation of a drug to a biomolecule.

### BACKGROUND OF THE INVENTION

Antibody-Drug Conjugates (ADC) are compounds wherein an antibody is covalently connected to a drug. This covalent connection is done via a linker. Therefore in the so called conjugation reaction, when an ADC is prepared, a drug, which is covalently modified by a linker, is covalently connected to the antibody. The covalent connection that is formed in the conjugation reaction is formed between the linker and the antibody. Often free thiol residues of the antibody are used for this covalent connection.
After the conjugation reaction, unreacted drug-linker is usually quenched by the addition of cysteine or N-acetylcysteine.
The residual unconjugated drug-linker, possibly quenched by the addition of cysteine or N-acetylcysteine, can be removed through purification on a size exclusion chromatography column (P. J. Carter et al., Mol. Cancer Ther. 2006, 5, 1474-1482).
The residual unconjugated drug-linker contributes to the so called unconjugated drug-related impurities, and the removal of these unconjugated drug-related impurities must be highly effective due to the high potency of the cytotoxic drugs used in ADCs.

One disadvantage of this method is that removal of drug-related impurities from the ADC solution is not efficient enough. Residual amounts of unconjugated drug-related impurities are typically still present in the ADC solution after purification.

There was a need for a method allowing a more efficient removal of unconjugated drug, unconjugated drug-linker and drug-related impurities after the conjugation reaction.

Unexpectedly the use of a solid-supported thiol residue as scavenger results in a method that removes unconjugated drug-related impurities more eficiently than the use of a size exclusion chromatography column.

### Abbreviations

- ADC: Antibody-Drug Conjugate
- PBS: phosphate-buffered saline
- vcMMAE: maleimidocaproyl-valine-citrulline-p-aminobenzyloxycarbonyl-monomethylauristatin E, CAS 646502-53-6

### SUMMARY OF THE INVENTION

Subject of the invention is the use of a compound THIOLSCAV for the removal of a compound DR after a reaction that covalently connects DR to a biomolecule;

THIOLSCAV is a solid support SOLSUPP, SOLSUPP has SH residues;
the SH residues are covalently connected to SOLSUPP via R1; or
the SH residues are covalently connected to SOLSUPP;
R1 are divalent connecting moieties covalently connecting a SH residue to SOLSUPP;

THIOLSCAV is represented by the formula (TS); 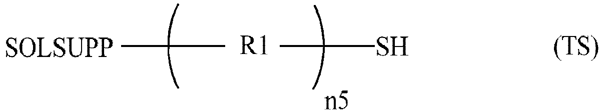 n5 is 0 or 1;
the SH residues are covalently connected to SOLSUPP via R1 in case of n5 being 1;
the SH residues are covalently connected to SOLSUPP in case of n5 being 0;
DR is a pharmaceutically active drug DRU covalently connected to a linker.

### DETAILED DESCRIPTION OF THE INVENTION

Further subject of the invention is a method for the removal of DR after a reaction that covalently connects DR to a biomolecule,
the method comprises a step STEP2;
in STEP2 a mixture MIXT is contacted with THIOLSCAV;
MIXT is obtained by the reaction that covalently connects DR to a biomolecule.

Further subject of the invention is said method comprising STEP2;
wherein the method comprises a further step, the step STEP1;
STEP1 comprises a reaction REAC-DR-BIOMOL between DR and a biomolecule;
REAC-DR-BIOMOL results in MIXT.

Preferably, after STEP2 a STEP3 is done, STEP3 comprises separating MIXT from THIOLSCAV.

THIOLSCAV is a thiol residue connected to SOLSUPP via R1 or directly. THIOLSCAV is known in the literature for its use as metal scavenger, for example for the removal of metal catalysts such as Pd after metal catalyzed reactions. THIOLSCAV is also known in the literature for its use in solid-phase synthesis.

Preferably, SOLSUPP is selected from the group consisting of silica, agarose, resin and organic polymer.
An organic polymer is preferably polystyrene based. The polystyrene can be crosslinked, e.g. with divinylbenzene.

Embodiments of SOLSUPP can have the form of fibres or beads. SOLSUPP can have a spherical or an irregular shape.

The residue -(R1)ₙ₅-SH of THIOLSCAV is called SCAV in the following, SCAV consists of the SH residue in case of n5 being 0, or of R1 and SH in case of n5 being 1.

Preferably, THIOLSCAV contains from 0.1 to 10 mmol, more preferably 0.1 to 5 mmol, of SCAV per g of THIOLSCAV; or
THIOLSCAV contains from 0.1 to 50 micromol, more preferably from 0.1 to 35 micromol, of SCAV per mL of THIOLSCAV.

Preferably, THIOLSCAV is selected from the group consisting of compound of formula (I), compound of formula (II), compound of formula (III), compound of formula (IV), compound of formula (V), compound of formula (VI), compound of formula (VII-1), compound of formula (VIII), compound of formula (X-1), compound of formula (XI-1), cysteine covalently connected to SOLSUPP, dithiothreitol covalently connected to SOLSUPP, and thionicotinamid covalently connected to SOLSUPP;

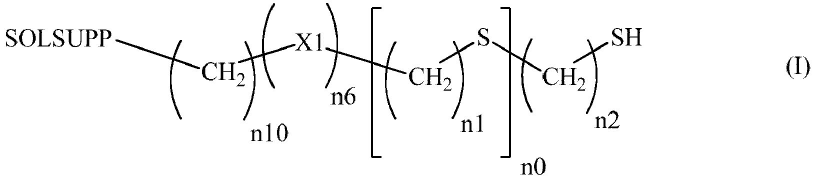

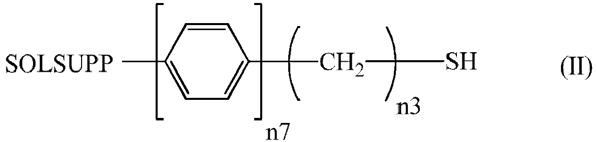

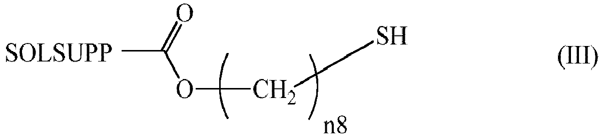

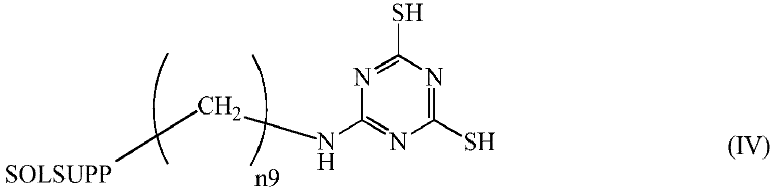

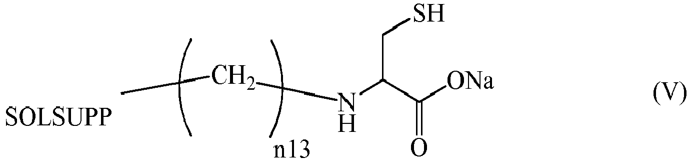

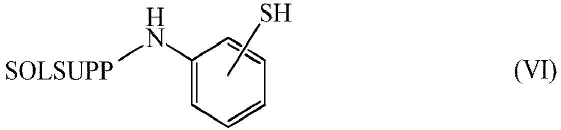

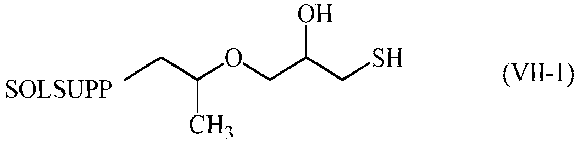

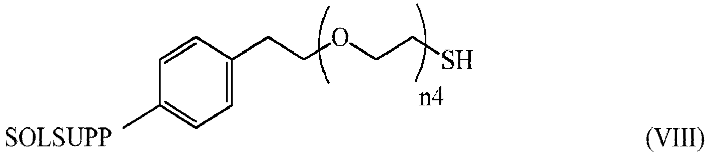

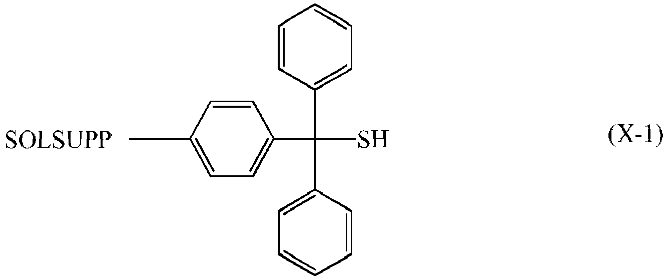

SOLSUPP -SH (XI-1)

- X1: is NH, O or S;
- n0: is 0, 1, 2, 3 or 4;
- n1: is 2 or 3;
- n2: is 2, 3 or 4;
- n3: is 0, 1 or 2;
- n4: is 0, 1, 2, 3, 4, 5, 6, 7, 8, 9, 10, 11, 12, 13, 14, 15, 16, 17, 18, 19, 20 or 21;
- n6: is 0 or 1;
- n7: is 0 or 1;
- n8: is 2, 3 or 4;
- n9: is 1, 2, 3 or 4;
- n10: is 0 or 1;
- n11: is 0 or 1;
- n12: is 0 or 1;
- n13: is 0 or 1.

Preferably,
- X1: is NH or O;
- n0: is 0 or 1;
- n1: is 2;
- n2: is 2 or 3;
- n3: is 1 or 2;
- n4: is 4, 5, 6, 9, 10, 11, 14, 15, 16, 17, 18, 19, 20 or 21
- n6: is 0 or 1;
- n7: is 1;
- n8: is 2;
- n9: is 3;
- n10: is 0 or 1;
- n11: is 0 or 1;
- n12: is 0 or 1;
- n13: is 0 or 1.

More preferably,
- X1: is NH or O;
- n0: is 0 or 1;
- n1: is 2;
- n2: is 2 or 3;
- n3: is 1 or 2;
- n4: is 5 or 10.
- n6: is 0 or 1;
- n7: is 1;
- n8: is 2;
- n9: is 3;
- n10: is 0 or 1;
- n11: is 1;
- n12: is 1;
- n13: is 1.

Preferably, the cysteine covalently connected to SOLSUPP is covalently connected to SOLSUPP via its NH₂-alpha residue or via its COOH-alpha residue.

Preferably, the dithiothreitol covalently connected to SOLSUPP is compound of formula (XII).

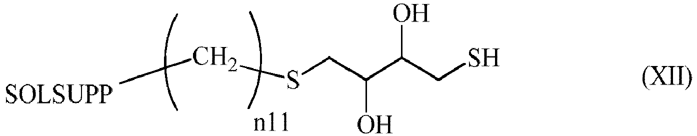

Preferably, the thionicotinamid covalently connected to SOLSUPP is compound of formula (XIII).

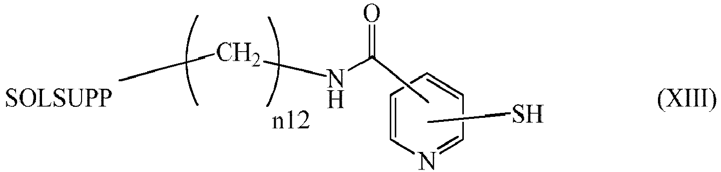

Preferably, R1 is selected from the group consisting of connecting moiety of formula (R1-1), connecting moiety of formula (R1-II), connecting moiety of formula (R1-III), connecting moiety of formula (R1-IV), connecting moiety of formula (R1-V), connecting moiety of formula (R1-VI), connecting moiety of formula (R1-VII-1), connecting moiety of formula (R1-VIII), connecting moiety of formula (R1-X-1), connecting moiety of formula (R1-XII), connecting moiety of formula (R1-CYS-C-1), connecting moiety of formula (R1-CYS-N-1), and connecting moiety of formula (R1-XIII); 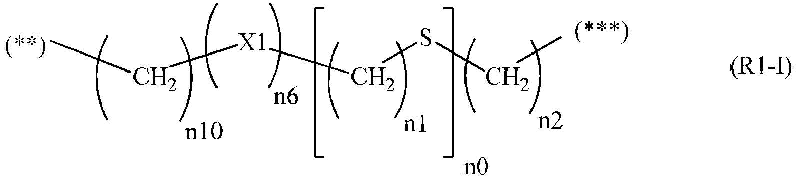 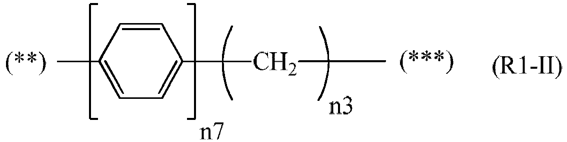 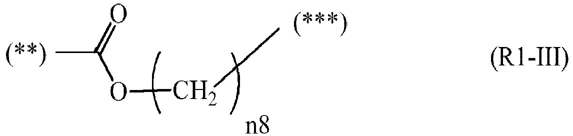 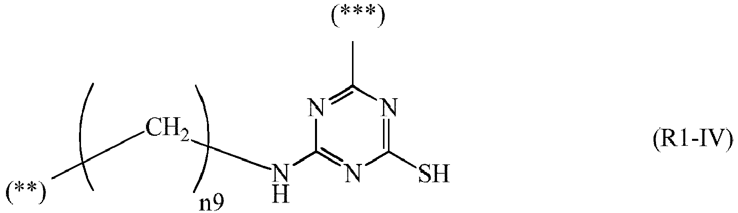 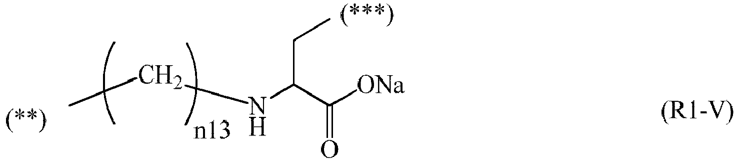 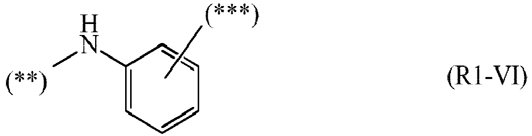 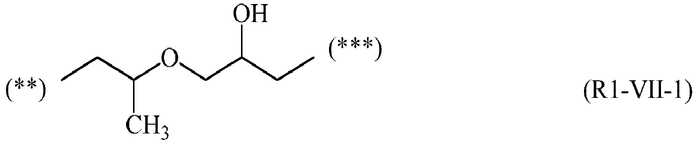 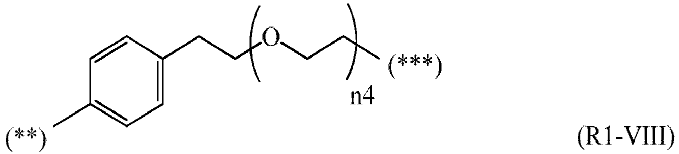 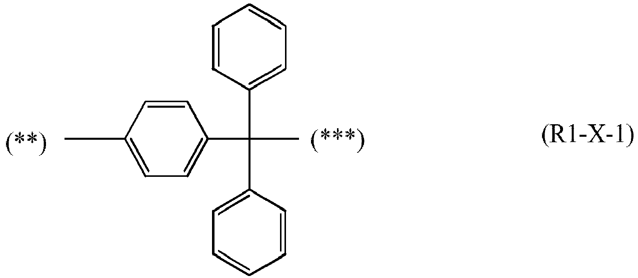 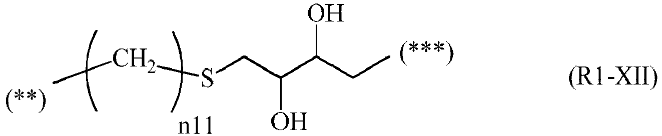 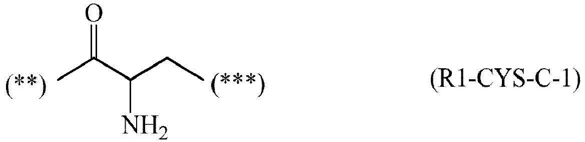 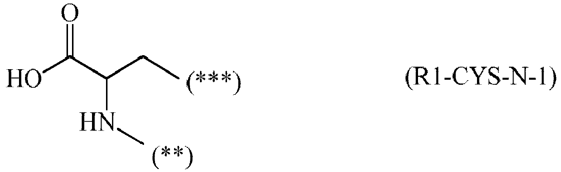 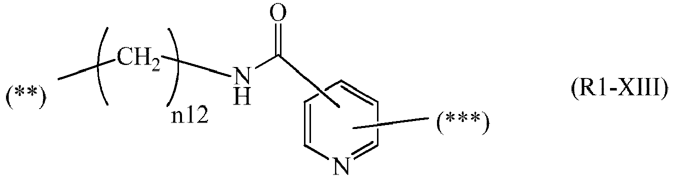 wherein
(**) denotes the covalent bond to SOLSUPP;
(***) denotes the covalent bond to the SH residue;
X1, n0, n1, n2, n3, n4, n6, n7, n8, n9, n10, n11 and n12 are as defined herein, also with all their embodiments.

Especially, THIOLSCAV is selected from the group consisting of compound of formula (1-1), compound of formula (1-2), compound of formula (1-3), compound of formula (1-4), compound of formula (1-5), compound of formula (1-6), compound of formula (1-7), compound of formula (II-1), compound of formula (II-2), compound of formula (III-1), compound of formula (IV-1), compound of formula (V-1), compound of formula (VI-1), compound of formula (VII-1), compound of formula (VIII-1), compound of formula (VIII-2), compound of formula (X-1), compound of formula (XI-1), compound of formula (XII-1), compound of formula (CYS-C-1), compound of formula (CYS-N-1), and compound of formula (XIII-1); 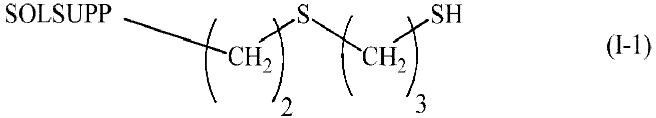 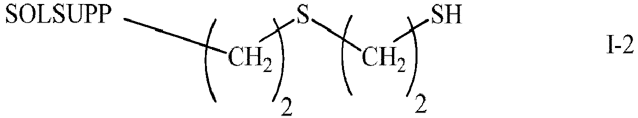 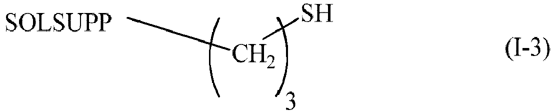 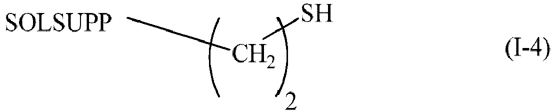 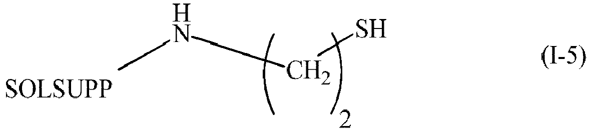 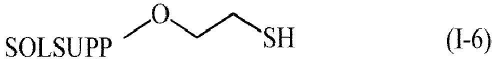 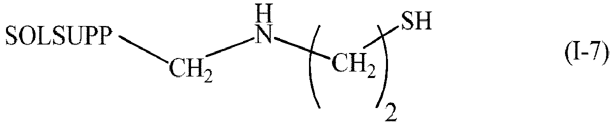 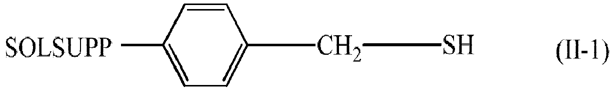 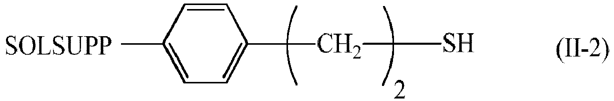 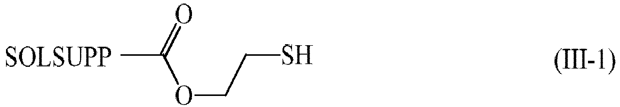 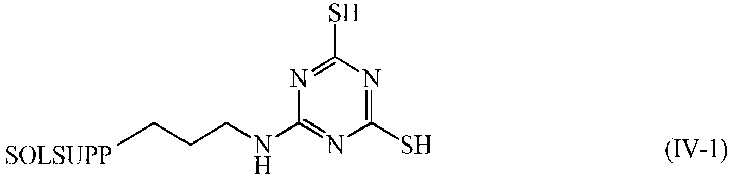 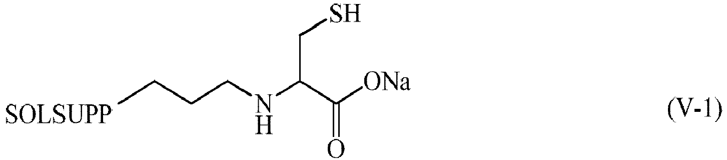 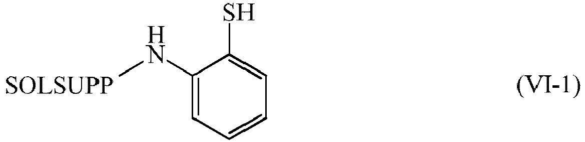 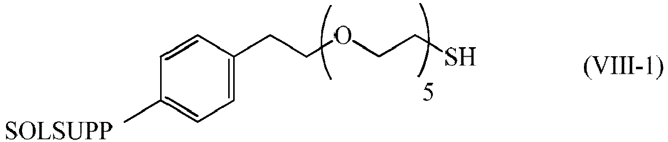 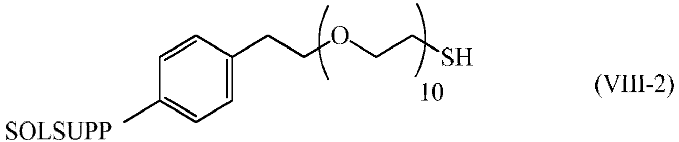 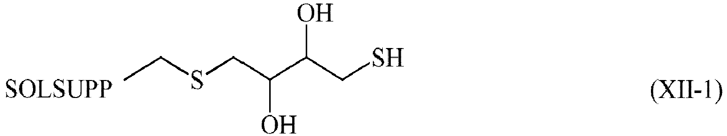 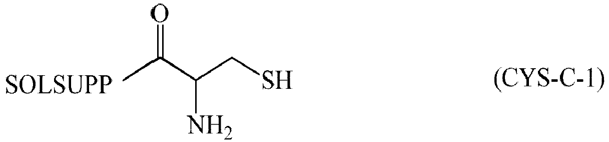 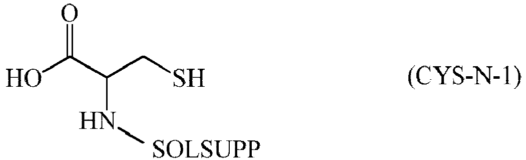 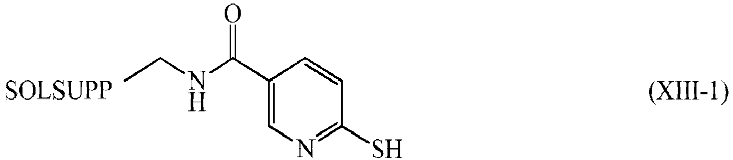 with
compound of formula (VII-1), compound of formula (X-1) and compound of formula (XI-1) as defined herein.

Preferably,
SOLSUPP is silica or polystyrene in case of compound of formula (I), compound of formula (II), compound of formula (IV), and compound of formula (V), compound of formula (VI), dithiothreitol covalently connected to SOLSUPP, and thionicotinamid covalently connected to SOLSUPP;
SOLSUPP is agarose in case of cysteine covalently connected to SOLSUPP;
more preferably,
SOLSUPP is silica in case of compound of formula (1-1), compound of formula (1-2), compound of formula (1-3), compound of formula (IV-1), and compound of formula (V-1);
SOLSUPP is polystyrene in case of compound of formula (1-4), compound of formula (1-5), compound of formula (1-7), compound of formula (II-1), compound of formula (II-2), compound of formula (VI-1), compound of formula (XII-1), and compound of formula (XIII-1);
SOLSUPP is agarose in case of cysteine covalently connected to SOLSUPP, compound of formula (CYS-C-1), and compound of formula (CYS-N-1).

Preferably, DR comprises a residue RESID, RESID is selected from the group consisting of iodoacetamide residue, bromoacetamide residue, maleimido residue, the maleimido residue being unsubstituted or substituted by 1 or 2 substitutents selected from the group consisting of Br and thiopyridyl, allene residue, arylpropionitrile residue, and sulfone residue.

The iodoacetamide residue has preferably the formula (IACRES);
the bromoacetamide residue has preferably the formula (BRACRES);
the allene residue has preferably the formula (ALLENRES);
the arylpropionitrile residue has preferably the formula (ARPROPNIRES);
the sulfone residue has preferably the formula (SULFONRES-I), the formula (SULFONRES-II) or the formula (SULFONRES-III); 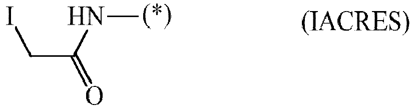 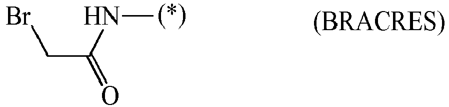 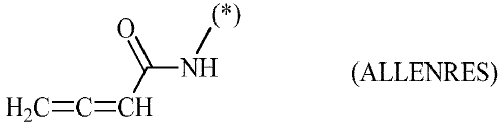 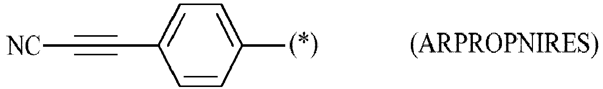 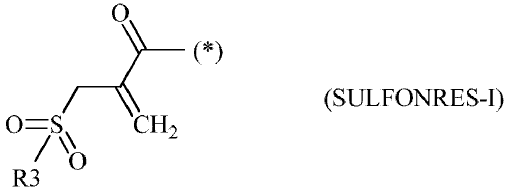 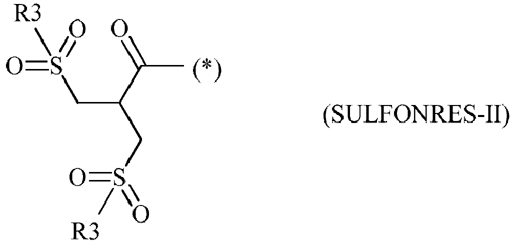 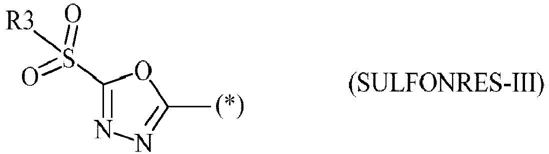 wherein (*) denotes the covalent bond from RESID to the remaining part of LIN;
R3 is C₁₋₄ alkyl, Ph or tolyl.

More preferably, RESID is selected from the group consisting of iodoacetamide residue, bromoacetamide residue, maleimido residue, the maleimido residue being unsubstituted or substituted by 2 identical substitutents selected from the group consisting of Br and thiopyridyl, allene residue, arylpropionitrile residue, and sulfone residue;
even more preferably, RESID has formula (BRACRES), formula (IACRES), formula (MALRES), formula (ALLENRES), formula (ARPROPNIRES), formula (SULFONRES-I), formula (SULFONRES-II) or formula (SULFONRES-III); 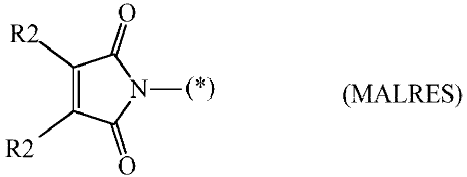 wherein (*) denotes the covalent bond from RESID to the remaining part of LIN; R2 is H, Br, S-C₁₋₁₂ alkyl, S-Ph or S-pyridyl;
with formula (BRACRES), formula (IACRES), formula (ALLENRES), formula (ARPROPNIRES), formula (SULFONRES-I), formula (SULFONRES-II) formula
(SULFONRES-III) as defined herein, also with all their embodiments; preferably R2 is S-Ph or S-pyridyl;
preferably the S-pyridyl is 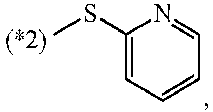 with (*2) denoting the bond to the 5-membered ring of the maleimido residue.

Even more preferably, RESID has formula (IACRES), formula (MRES), formula (BRMRES), formula (THIOPYRMRES), or formula (SULFONRES).

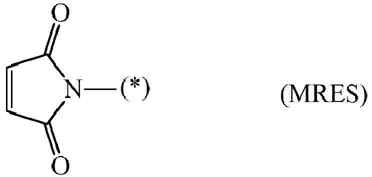

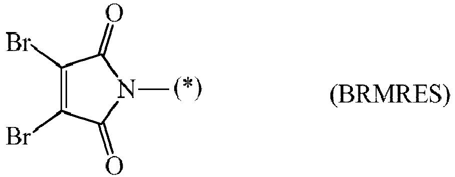

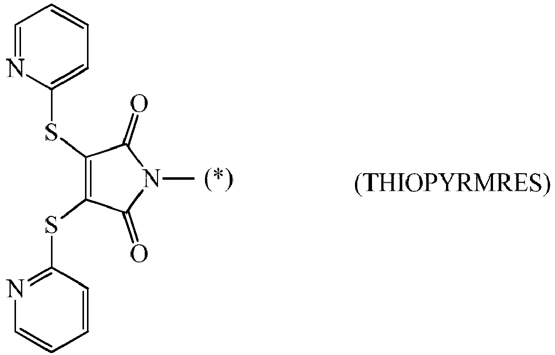

Especially, RESID is is selected from the group consisting of iodoacetamide residue, maleimido residue, the maleimido residue being unsubstituted or substituted be 1 or 2 substitutents selected from the group consisting of Br and thiopyridyl, and sulfone residue;
more especially, RESID is selected from the group consisting of iodoacetamide residue, maleimido residue, the maleimido residue being unsubstituted or substituted be 2 identical substitutents selected from the group consisting of Br and thiopyridyl, and sulfone residue;
even more especially, RESID is selected from the group consisting of maleimido residue, the maleimido residue being unsubstituted or substituted be 2 identical substitutents selected from the group consisting of Br and thiopyridyl;
in particular, RESID has the formula (MRES).

Preferably, it is the linker that comprises RESID, also with all the embodiments of RESID. The linker is called LIN herein.

LIN can be any linker that is used for conjugating a biolomolecule to a drug. LIN is known in the literature, for example L. Ducry et al., Bioconjugate Chem. 2010, 21, 5-13, or N. Jain et al., Pharm. Res. 2015, doi:10.1007/s11095-015-1657-7.

Preferably, LIN is a linker that is used to conjugate an antibody to a drug in order to obtain an ADC.
Typical components, from which LIN is build, are amino acids and spacer groups, without limiting the invention to these components.
Amino acids are typically selected from the group consisting of naturally occuring amino acids, H₂N-(CH₂)ₙ₁₄-COOH and H₂N-CH₂-cyclohexane-COOH;
n14 is 2, 3, 4, 5, 6, 7, 8, 9 or 10;
preferably, n14 is 3, 4, 5 or 6;
more preferably, n14 is 3, 4 or 5;
even more preferably, n14 is 3 or 5;
especially, n14 is 5.
Naturally occuring amino acids are preferably selected from the group consisting of valine, citrulline, phenylalanine and alanine.
The amino acids are typically covalently connected in LIN to other components of LIN, to RESID and/or to DRU via their amino residue and their COOH residue.
The N atom, that is displayed in some of the formulae of RESID and that is marked with the (*) and is connected to the remaining part of LIN, can stem from the other components of LIN.

Spacer groups are typically based on ethylene oxide, aminobenzylalcohol, ethylenedamine, N-methylethylenediamine, N,N'-dimethylethylenediamine, carbonyl, hydrazide, or triazole.
Typical components, from which LIN is build, have formulae such as CH₂-CH₂-O, HN-Ph-CH₂-O, N-Ph-CH₂-O, HN-(CH₂)ₙ₁₄-COOH, N-(CH₂)ₙ₁₄-COOH, HN-CH₂-cyclohexane-C(O), N- CH₂-cyclohexane-C(O), N(CH₃)-CH₂-CH₂-N(CH₃), CO and NH-N.

Preferably, the aminobenzylalcohol is p-aminobenzylalcohol.
Preferably, the cyclohexane derived amino acid has para substitution with regard to the aminomethylene residue and the C(O) residue.

Preferably, DR is selected from the group consisting of compound of formula (DR-I), compound of formula (DR-II), compound of formula (DR-III), compound of formula (DR-IV), compound of formula (DR-V), compound of formula (DR-VI), compound of formula (DR-VII), compound of formula (DR-VIII), compound of formula (DR-IX), compound of formula (DR-X), compound of formula (DR-XI), compound of formula (DR-XII) and compound of formula (DR-XIII); 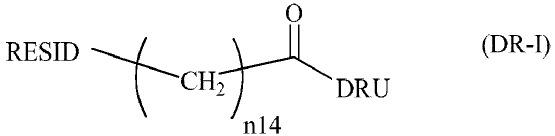 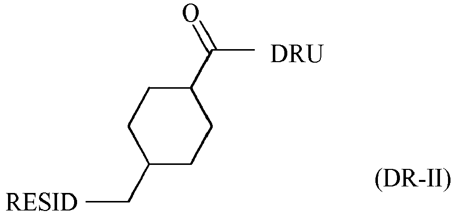 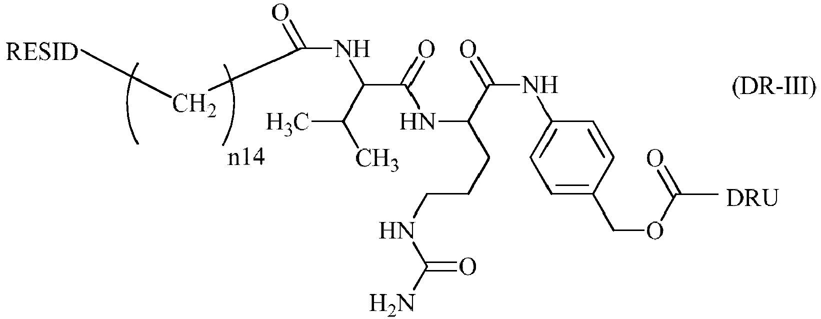 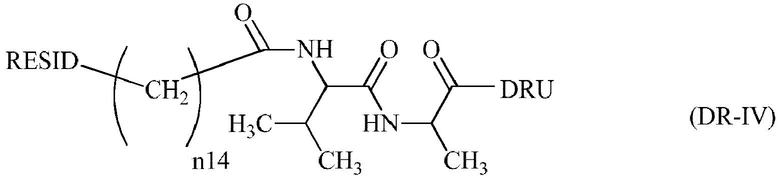 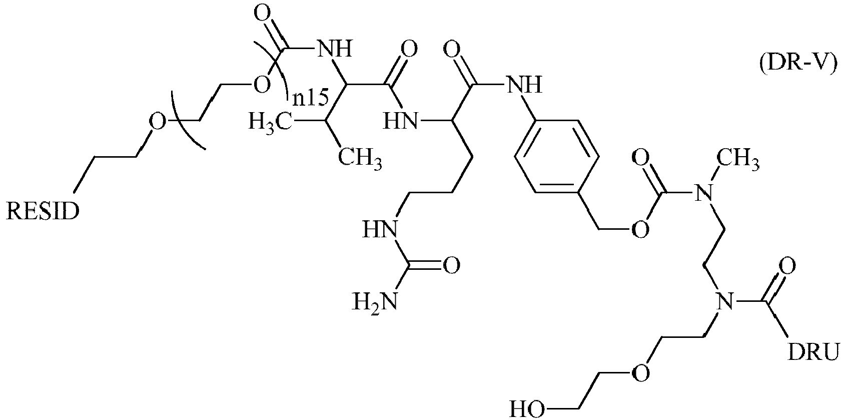 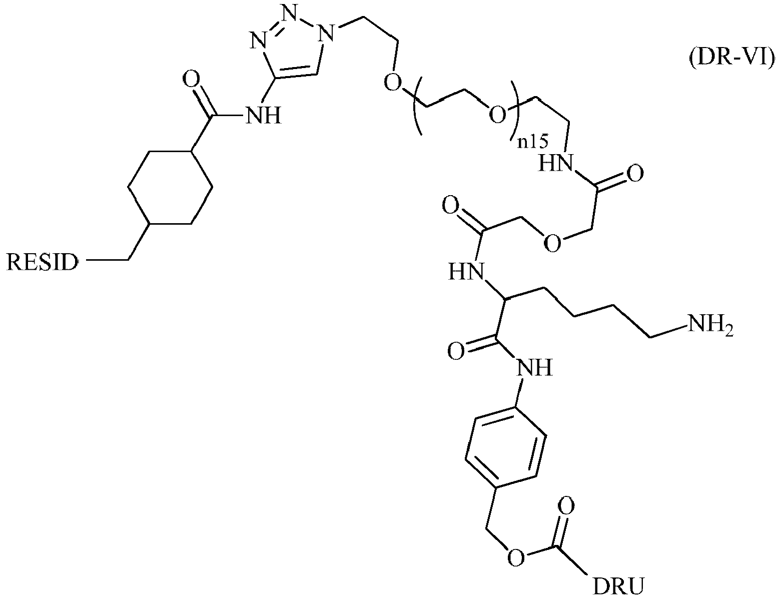 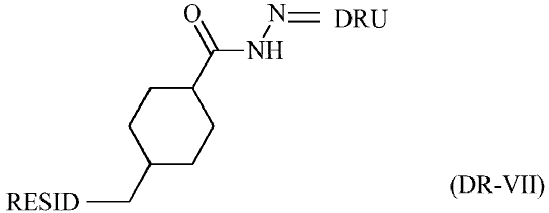 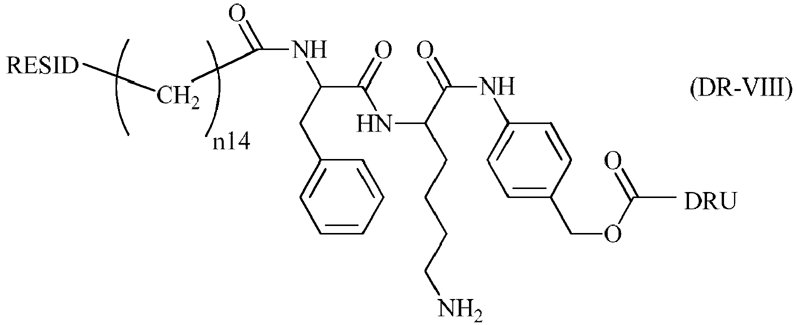 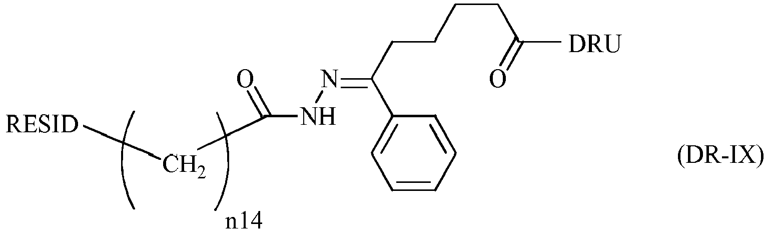 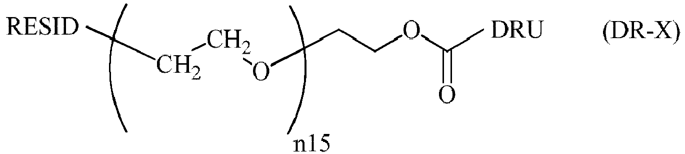 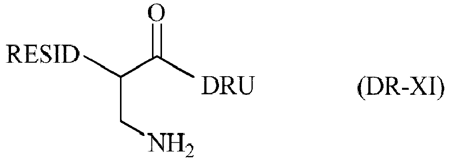 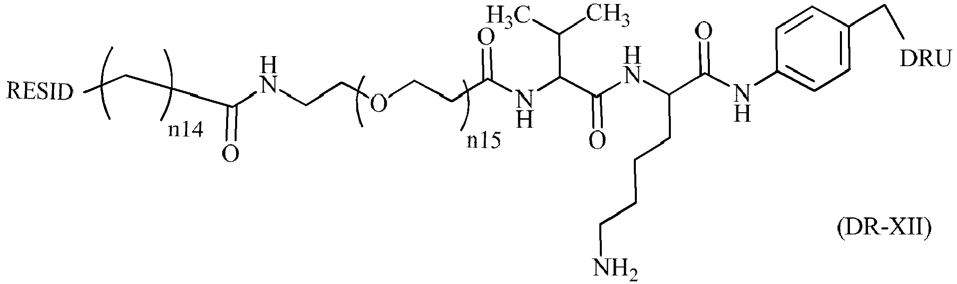 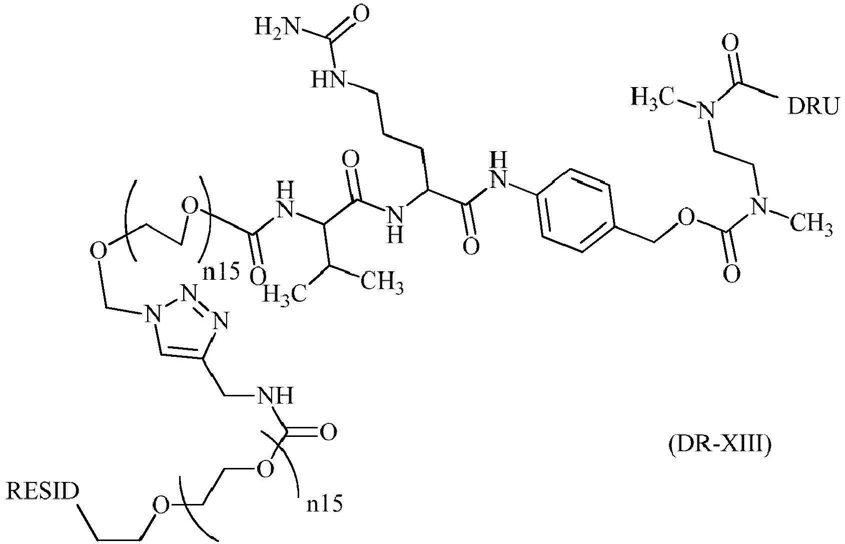 wherein
DRU, RESID and n14 are as defined herein, also with all their embodiments;
n15 is 0, 1, 2, 3, 4, 5, 6, 7, 8, 9 or 10;
preferably, n15 is 1, 4, 5 or 7;
more preferably,
n15 is 1 in case of compound of formula (DR-V),
n15 is 7 in case of compound of formula (DR-VI),
n15 is 1 in case of compound of formula (DR-X),
n15 is 4 in case of compound of formula (DR-XII) and
n15 is 1 in case of compound of formula (DR-XIII).
more prefererably, DR is selected from the group consisting of compound of formula (DR-M-I), compound of formula (DR-II), compound of formula (DR-III) and compound of formula (DR-IV).

Preferably, the biomolecule is a compound BIOMOL, and BIOLMOL is a protein;
more preferably, BIOMOL is selected from the group consisting of monoclonal antibody, polyclonal antibody, antibody fragment, protein and peptide;
even more preferably, BIOMOL is a monoclonal antibody.

Preferably, DRU is selected from the group consisting of cytotoxic agents, chemotherapeutic and antimetastatic agents.

Preferably, the chemotherapeutic and antimetastatic agents are selected from the group consisting of tyrosine kinase inhibitors and Rac1 inhibitors.

Preferably, tyrosine kinase inhibitors are selected from the group of active pharmaceutical ingredients (API) consisting of Imatinib, Lapatimib, Sunitimib, Nilotimib and Dasatimib.

Preferably, Rac1 inhibitors is NSC 23766.

Preferable cytotoxic agents are those used for cancer therapy.
Preferable classes of cytotoxic agents include, for example, the enzyme inhibitors such as the anthracycline family of drugs, the bleomycins, the cytotoxic nucleosides, dihydrofolate reductase inhibitors, differentiation inducers, DNA cleavers, DNA intercalators, DNA alkylating agents, diynenes, the mitomycins, the podophyllotoxins, the pteridine family of drugs, taxols, thymidylate synthase inhibitors, topoisomerase inhibitors, and the vinca drugs.
Preferable useful members of various classes of cytotoxic agents are selected from the group consisting of N8-acetyl spermidine, actinomycin, 9-amino camptothecin, aminopterin, anguidine, anthracycline, auristatin, benzodiazepine, bleomycin, calicheamycin, camptothecin (lactone or ring-opened form of the lactone), carminomycin, CC-1065, clofaribine, 1-(2-chloroethyl)-1,2-dimethanesulfonyl hydrazide, cyclopropabenzindol-4-one (CBI), cytarabine, cytosine arabinoside, daunorubicin, dichloromethotrexate, n-(5,5-diacetoxy-pentyl) doxorubicin, 1,8-dihydroxy-bicyclo[7.3.1] trideca-4-9-diene-2,6-diyne-13-one, difluoronucleosides, doxorubicin, duocarmycin, epirubicin, esperamicin, etoposide, 5-fluorouracil, irinotecan, leurosideine, leurosine, maytansine, melphalan, 6-mercaptopurine, methopterin, methotrexate, mitomycin A, mitomycin C, morpholine-doxorubicin, nemorubicin, podophyllotoxin and podophyllotoxin derivatives such as etoposide or etoposide phosphate, retinoic acid, saporin, tallysomycin, vinblastine, vincristine, vindesine, taxane, such as taxol or paclitaxel, taxotere or docetaxel, and taxotere retinoic acid, and analogues and derivatives thereof.

More preferable cytotoxic agents are selected from the group consisting of anthracycline, auristatin, benzodiazepine, calicheamycin, cyclopropabenzindol-4-one (CBI), doxorubicin, duocarmycin, maytansine, mitomycin C, taxol, and analogues and derivatives of these substances.

THIOLSCAV are commercially available products, e.g. from PhosphonicS Ltd, UK, under section Pharmaceuticals; Johnson Matthey Plc, UK, tradenames Smopex®, QuadraPur®, QuadrSil®; SiliCycle Inc., CA, under tradename SiliaMetS®; Thermo Fisher Scientific Inc., US, under tradename Pierce®; SIGMA-ALDRICH Co., US, under tradenames QuadraSil®, QuadraPure®, and under sections MetalScavenger, Scavenger, Solid Supported Synthesis; Iris Biotech GmbH, DE; Bachem Holding AG, CH; or can be prepared according to known methods. vcMMAE is commercially available, e.g from Concortis Biosystems, Inc., USA, or from AstaTech, Inc., USA, and can be prepared according to known methods.

### EXAMPLES

### Methods

### HIC-HPLC: Hydrophobic Interaction Chromatography HPLC

TSK-gel® Butyl-NPR 4.6 mm x 35 mm column (NPR means non-porous resin, polymethacrylate base material with mean particle size 2,5 micrometer, purchased from Tosoh Bioscience LLC, US), solvent A: 50 mM sodium phosphate buffer, solvent B: 25% (v/v) isopropanol in 50 mM sodium phosphate buffer, gradient from 100% solvent A to 100% solvent B over 12 min., 0.8 ml/min, detection at 280 nm.

### RP-HPLC: Reversed Phase HPLC

Xterra® C18, 2.1 x 100 mm, 3.5 µm (purchased from Waters Corporation, US), solvent A: 0.1 % (v/v) TFA in water, solvent B: 0.1 % (v/v) TFA in acetonitrile, gradient from 25 to 100% solvent B over 28 min., 0.3-0.6 ml/min., detection at 214 nm.

### SEC-HPLC: Size Exclusion Chromatography

TSKgel® G3000SWXL 300 x 7.8 mm column (Silica based column with mean pore size of 250 Angstrom and mean particle size of 5 micrometer, purchased from Tosoh Bioscience LLC, US), eluent 10% (v/v) isopropanol in 0.2 M potassium phosphate buffer, 0.5 ml/min., detection at 280 nm.

### Sample Handling

Samples are analyzed straight after they have been taken, or, if there is a delay, they are stored at-80 °C in between

### Materials

- NAP-25 column: GE Healthcare, 17-0852-02, pack of 50 columns
- SPM32d column: 3-mercaptopropyl ethyl sulfide silica, 1 g cartridge from PhosphonicS Ltd, UK, containing 1.0 mmol thiol-functional residue/g
- IgG1 antibody solution: from Lonza Ltd, CH, aqueous buffer of 4.38 mM histidine / histidine HCl, 150 mM trehalose, 0.0088% (w/v) polysorbate 20, pH 5.9

### Example 1

The pH of an IgG1 antibody solution (5.0 ml, 0.86 micromol) was adjusted to 7.5 with a 0.1 M aq. NaOH solution. A 10 mM vcMMAE solution in N,N-dimethylacetamide (290 microliter; 2.90 micromol; 3.4 eq.) was added and the solution was stirred for 2 min. at 20 °C.
- A sample No 1 was taken and was analyzed by RP-HPLC.
- The remaining solution was filtered over a SPM32d column. A sample No 2 was taken and was analyzed by RP-HPLC.
- The remaining solution was filtered over the same SPM32d column. A sample No 3 was taken and was analyzed by RP-HPLC.
- The remaining solution was filtered over a SPM32d column. A sample No 4 was taken and was analyzed by RP-HPLC.

| **Sample No** | **Filtrations** | **vcMMAE** |
|---|---|---|
| | | **[micromol** / **liter**] |
| **1** | 0 | 266.385 |
| **2** | 1 | 0.066 |
| **3** | 2 | 0.003 |
| **4** | 3 | < 0.001 |

### Example 2

An IgG1 antibody solution (2.0 ml; 0.32 micromol) was diluted with PBS buffer (2.0 ml) and the pH of the solution adjusted to 7.5 with a 0.5 M aq. Na₂HPO₄ solution. A 10 mM aqueous tris(2-carboxyethyl)phosphine hydrochloride solution (Sigma-Aldrich C4706, 70 microliter; 0.70 micromol; 2.2 eq.) was added and the solution was stirred for 90 min. at 20 °C. A 10 mM vcMMAE solution in N,N-dimethylacetamide (190 microliter; 1.90 micormol; 6 eq.) was added and the solution was stirred for 30 min. at 20 °C. At this stage, the reaction mixture was split into two equal portions, portion A and portion B.

### Comparative Example 2a, standard purification technique:

To portion A, a 10 mM N-acetyl-cysteine solution in N,N-dimethylacetamide (207 microliter; 2.07 micromol; 13 eq.) was added and the solution stirred for 30 min. at 20 °C. The reaction mixture was purified over a NAP-25 column, eluting once with PBS buffer. The ADC-containing fractions were pooled and analyzed:
- purity (SEC-HPLC): 98.7 area%
- drug-to-antibody ratio (HIC-HPLC): 4.2
- N-acetyl-cystein-vcMMAE conjugate (RP-HPLC): 0.538 micromol / liter
- unconjugated vcMMAE (RP-HPLC): 0.004 micromol / liter

### Example 2b, purification according to invention

Portion B was filtered three times over a SPM32d column and was then purified over a NAP-25 column, eluting once with PBS buffer. The ADC-containing fractions were pooled and analyzed:
- purity (SEC-HPLC): > 99.5 area%.
- drug-to-antibody ratio (HIC-HPLC): 4.1
- N-acetyl-cystein-vcMMAE (RP-HPLC): < 0.001 micromol / liter
- unconjugated vcMMAE (RP-HPLC): < 0.001 micromol / liter

The results show that the standard treatment with N-acetyl-cysteine does not remove residual free MMAE species as effectively as the purification according to invention.

## Claims

1. Use of a compound THIOLSCAV for the removal of a compound DR after a reaction that covalently connects DR to a biomolecule;
THIOLSCAV is a solid support SOLSUPP, SOLSUPP has SH residues;
the SH residues are covalently connected to SOLSUPP via R1; or
the SH residues are covalently connected to SOLSUPP;
R1 are divalent connecting moieties covalently connecting a SH residue to SOLSUPP;
THIOLSCAV is represented by the formula (TS); 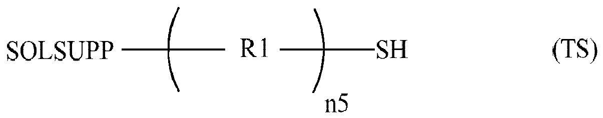 n5 is 0 or 1;
the SH residues are covalently connected to SOLSUPP via R1 in case of n5 being 1;
the SH residues are covalently connected to SOLSUPP in case of n5 being 0;
DR is a pharmaceutically active drug DRU covalently connected to a linker.

2. Use according to claim 1, wherein
DR comprises a residue RESID, RESID is selected from the group consisting of iodoacetamide residue, bromoacetamide residue, maleimido residue, the maleimido residue being unsubstituted or substituted by 1 or 2 substitutents selected from the group consisting of Br and thiopyridyl, allene residue, arylpropionitrile residue, and sulfone residue.

3. Use according to claim 2, wherein
the iodoacetamide residue has preferably the formula (IACRES);
the bromoacetamide residue has preferably the formula (BRACRES);
the allene residue has preferably the formula (ALLENRES);
the arylpropionitrile residue has preferably the formula (ARPROPNIRES);
the sulfone residue has preferably the formula (SULFONRES-I), the formula (SULFONRES-II) or the formula (SULFONRES-III); 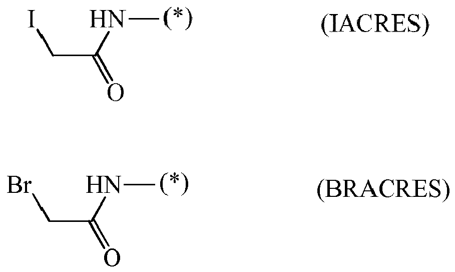 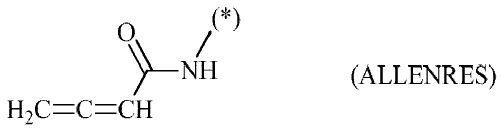 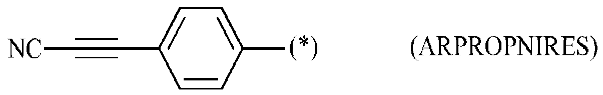 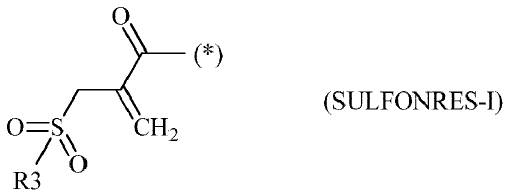 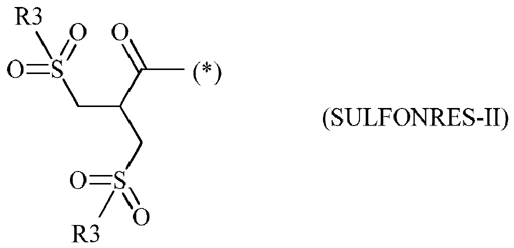 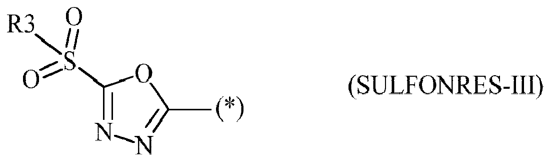 wherein (*) denotes the covalent bond from RESID to the remaining part of LIN;
R3 is C₁₋₄ alkyl, Ph or tolyl.

4. Use according to claim 2 or 3, wherein
RESID has formula (BRACRES), formula (IACRES), formula (MALRES), formula (ALLENRES), formula (ARPROPNIRES), formula (SULFONRES-I), formula (SULFONRES-II) or formula (SULFONRES-III); 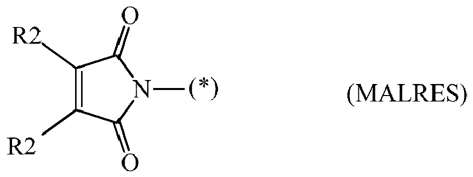 R2 is H, Br, S-C₁₋₁₂ alkyl, S-Ph or S-pyridyl;
with formula (BRACRES), formula (IACRES), formula (ALLENRES), formula (ARPROPNIRES), formula (SULFONRES-I), formula (SULFONRES-II) formula (SULFONRES-III) as defined in claim 3.

5. Use according to one or more of claims 2 to 4, wherein
the linker comprises RESID.

6. Use according to one or more of claims 1 to 5, wherein
the biomolecule is a compound BIOMOL, and BIOLMOL is a protein.

7. Use according to one or more of claims 1 to 6, wherein
BIOMOL is selected from the group consisting of monoclonal antibody, polyclonal antibody, antibody fragment, protein and peptide.

8. Use according to one or more of claims 1 to 7, wherein
BIOMOL is a monoclonal antibody.

9. Use according to one or more of claims 1 to 8, wherein
SOLSUPP is selected from the group consisting of silica, agarose, resin and organic polymer.

10. Use according to one or more of claims 1 to 9, wherein
THIOLSCAV is selected from the group consisting of compound of formula (I), compound of formula (II), compound of formula (III), compound of formula (IV), compound of formula (V), compound of formula (VI), compound of formula (VII-1), compound of formula (VIII), compound of formula (X-1), compound of formula (XI-1), cysteine covalently connected to SOLSUPP, dithiothreitol covalently connected to SOLSUPP, and thionicotinamid covalently connected to SOLSUPP; 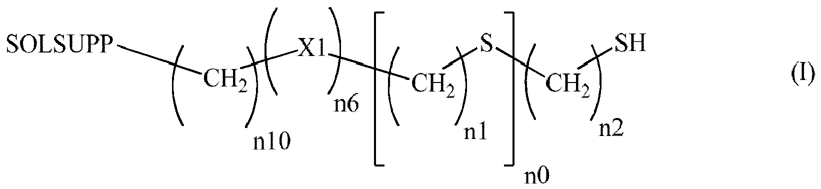 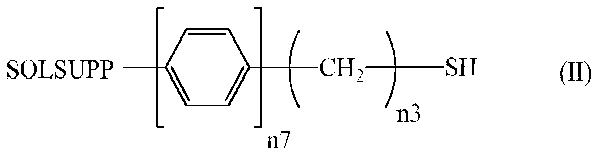 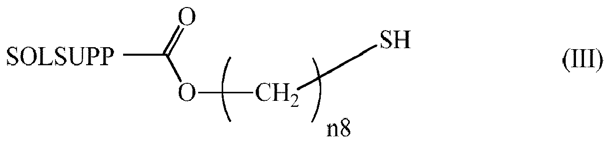 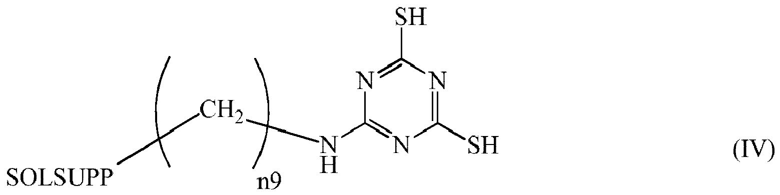 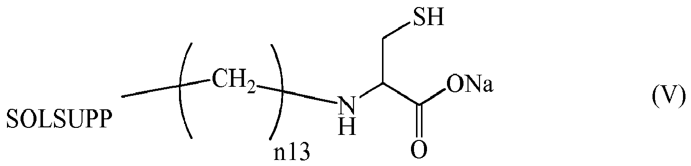 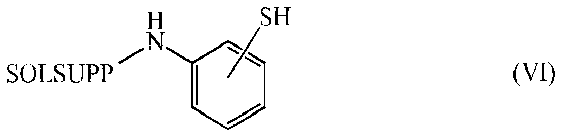 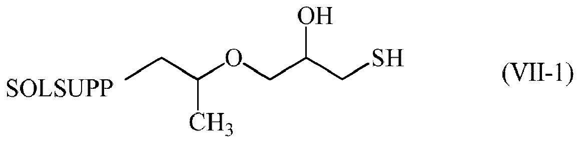 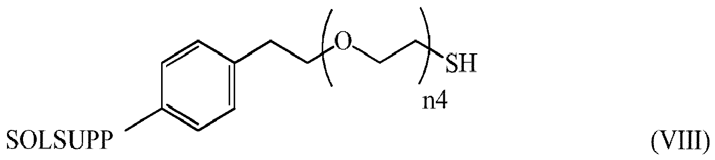 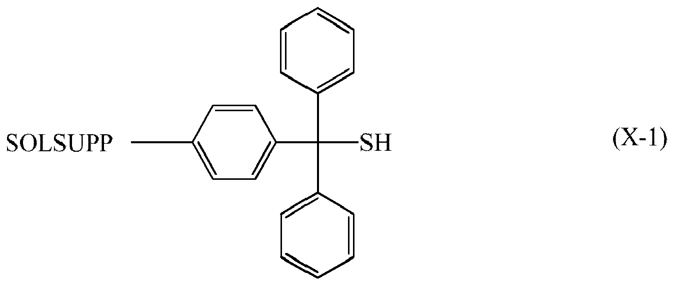
SOLSUPP -SH (XI-1)
X1 is NH, O or S;
n0 is 0, 1, 2, 3 or 4;
n1 is 2 or 3;
n2 is 2, 3 or 4;
n3 is 0, 1 or 2;
n4 is 0, 1, 2, 3, 4, 5, 6, 7, 8, 9, 10, 11, 12, 13, 14, 15, 16, 17, 18, 19, 20 or 21;
n6 is 0 or 1;
n7 is 0 or 1;
n8 is 2, 3 or 4;
n9 is 1, 2, 3 or 4;
n10 is 0 or 1;
n11 is 0 or 1;
n12 is 0 or 1;
n13 is 0 or 1.

11. Use according to claim 10, wherein
X1 is NH or O;
n0 is 0 or 1;
n1 is 2;
n2 is 2 or 3;
n3 is 1 or 2;
n4 is 4, 5, 6, 9, 10, 11, 14, 15, 16, 17, 18, 19, 20 or 21
n6 is 0 or 1;
n7 is 1;
n8 is 2;
n9 is 3;
n10 is 0 or 1;
n11 is 0 or 1;
n12 is 0 or 1;
n13 is 0 or 1.

12. Use according to claim 10 or 11, wherein
the dithiothreitol covalently connected to SOLSUPP is compound of formula (XII).

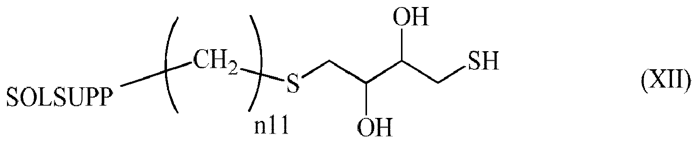

13. Use according to one or more of claims 10 to 12, wherein
the thionicotinamid covalently connected to SOLSUPP is compound of formula (XIII).

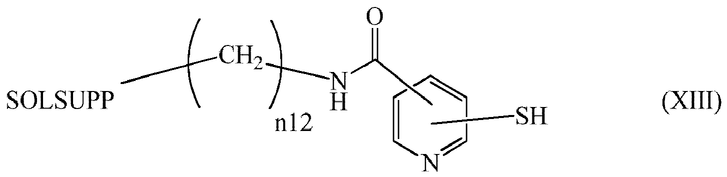

14. Use according to one or more of claims 1 to 13, wherein
THIOLSCAV is selected from the group consisting of compound of formula (1-1), compound of formula (1-2), compound of formula (1-3), compound of formula (1-4), compound of formula (I-5), compound of formula (1-6), compound of formula (1-7), compound of formula (II-1), compound of formula (II-2), compound of formula (III-1), compound of formula (IV-1), compound of formula (V-1), compound of formula (VI-1), compound of formula (VII-1), compound of formula (VIII-1), compound of formula (VIII-2), compound of formula (X-1), compound of formula (XI-1), compound of formula (XII-1), compound of formula (CYS-C-1), compound of formula (CYS-N-1), and compound of formula (XIII-1); 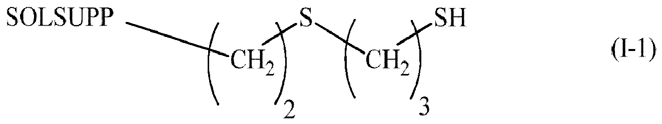 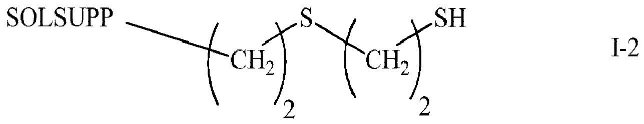 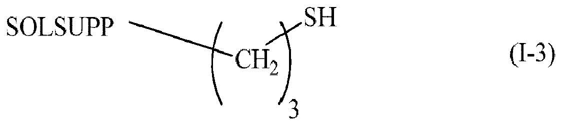 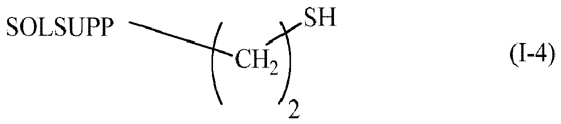 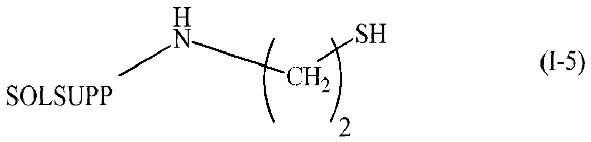 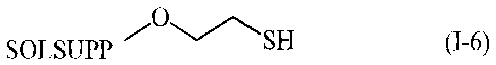 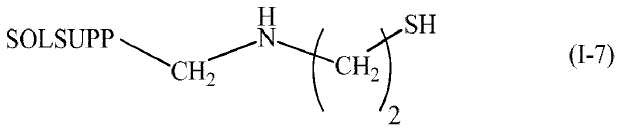 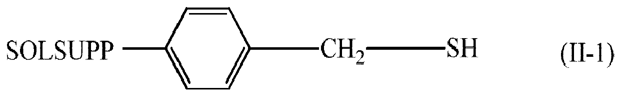 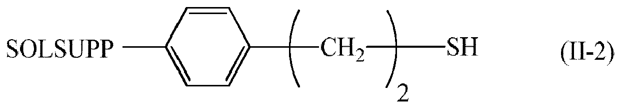 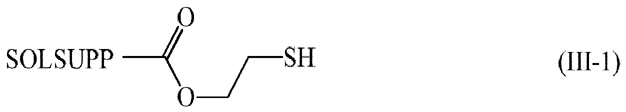 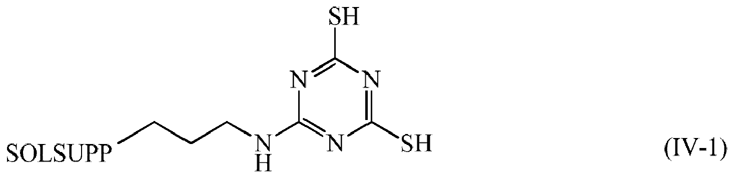 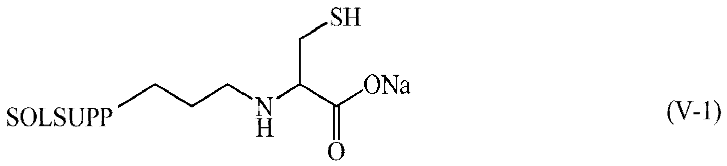 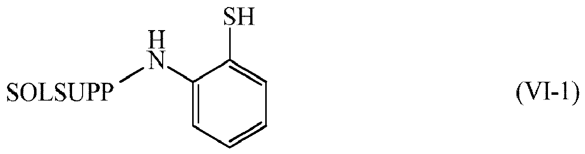 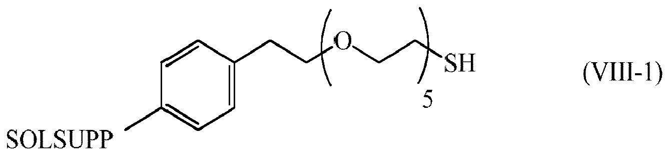 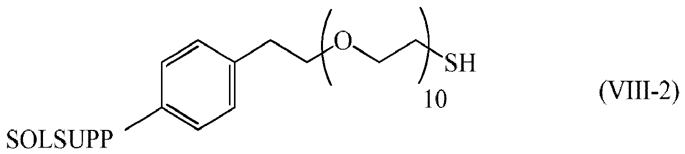 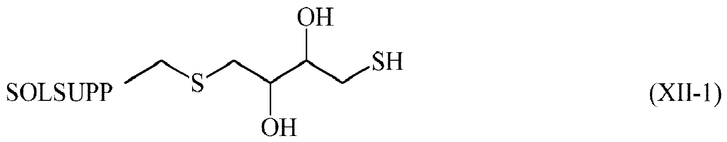 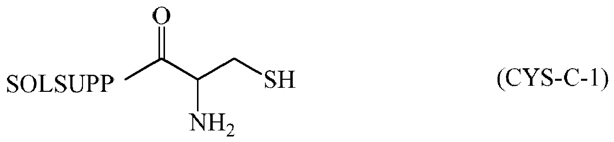 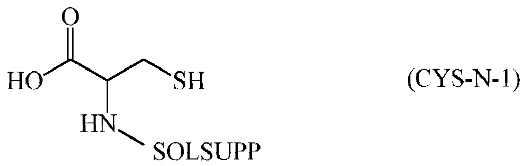 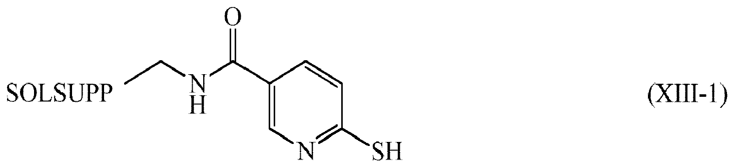 with
compound of formula (VII-1), compound of formula (X-1) and compound of formula (XI-1) as defined in claim 10.

15. Use according to one or more of claims 10 to 14, wherein
SOLSUPP is silica or polystyrene in case of compound of formula (I), compound of formula (II), compound of formula (IV), and compound of formula (V), compound of formula (VI), dithiothreitol covalently connected to SOLSUPP, and thionicotinamid covalently connected to SOLSUPP;
SOLSUPP is agarose in case of cysteine covalently connected to SOLSUPP.
